# EUROPEAN PATENT APPLICATION

(11) **EP 4 782 008 A1**
(43) Date of publication of application: **29.07.2026**
(21) Application number: 24867416.0
(22) Date of filing: 18.09.2024
(51) Int. Cl.: A61K 47/38, A61K 31/445, A61P 25/28, A61P 25/16, A61P 25/08, A61P 25/18, A61K 47/36, A61K 47/12, A61K 9/20

(54) **PHARMACEUTICAL COMPOSITION OF FLUORINE-SUBSTITUTED CYCLIC AMINE COMPOUND OR SALT THEREOF, AND PREPARATION METHOD THEREFOR**

(30) Priority: 18.09.2023 CN 202311206152
(71) Applicant: Jiangsu Kanion Pharmaceutical Co., Ltd., Lianyungang, Jiangsu 222047 (CN)
(72) Inventor: XIAO, Wei, Lianyungang, Jiangsu 222047 (CN); ZHANG, Dayong, Lianyungang, Jiangsu 222047 (CN); HE, Xiaolian, Lianyungang, Jiangsu 222047 (CN); WANG, Xue, Lianyungang, Jiangsu 222047 (CN); SUN, Xia, Lianyungang, Jiangsu 222047 (CN); WANG, Tuanjie, Lianyungang, Jiangsu 222047 (CN); WANG, Zhenzhong, Lianyungang, Jiangsu 222047 (CN); GUO, Qingming, Lianyungang, Jiangsu 222047 (CN)
(74) Representative: Boco IP Oy Ab
(86) International application number: PCT/CN2024/119304
(87) International publication number: WO 2025/061008

(57) **Abstract**

The present application relates to a pharmaceutical composition and preparation of a compound represented by formula (I) or a salt thereof, and a preparation method therefor. Specifically, the present application discloses a pharmaceutical composition using the compound represented by formula (I) or a pharmaceutically acceptable salt thereof as an active ingredient. The pharmaceutical composition contains the compound represented by formula (I) or a pharmaceutically acceptable salt thereof, and a pharmaceutically acceptable excipient. The pharmaceutical composition of the present application has good drug stability and safety, involves a simple and convenient preparation method, and is suitable for industrial production.

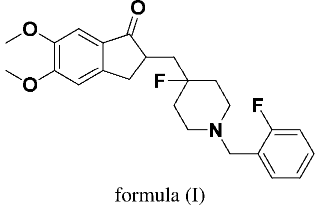

## Description

### CROSS-REFERENCE TO RELATED APPLICATIONS

The present application claims priority to and the benefit of Chinese Patent Application No. 202311206152.2, filed with China National Intellectual Property Administration on September 18, 2023, the contents of which are incorporated herein by reference in their entirety.

### FIELD OF THE INVENTION

The present application belongs to the field of pharmaceutical preparations, and in particular, the present application relates to a pharmaceutical composition of an indanone compound, a preparation thereof, and a preparation method therefor.

### BACKGROUND

Alzheimer's disease (AD) is a fatal primary neurodegenerative disease of the central nervous system and the most common type of senile dementia. Acetylcholinesterase inhibitors (AChEIs) are currently the main drugs for the treatment of AD. AChE inhibitors can improve cognitive function and enhance abilities of memory and thinking by inhibiting cholinesterase activity, increasing acetylcholine neurotransmitters in the cerebral cortex and hippocampus, and ameliorating neuro-neuronal synaptic dysfunction as well as the reduction of acetylcholine neurotransmitters caused by cell death or functional degeneration. The currently marketed AChE inhibitors are mainly donepezil, huperzine A, and galanthamine. According to the survey, among the major varieties of anti-dementia drugs in sample hospitals across 22 cities in China, the AChE inhibitor donepezil of Japan's Eisai Co., Ltd. represented nearly 57% of the market share; while the natural product drug huperzine A, which was independently researched and developed in China, only represented 25% of the market share.

CN103787954A discloses 2-(4-fluoro-(1-(2-fluorobenzyl))piperidin-4-yl-methyl)-5,6-dimethoxy-2,3-dihydro-1-indanone (hereinafter abbreviated as the compound of formula (I)), the specific structure thereof is as follows. It is a novel and highly effective acetylcholinesterase inhibitor with high affinity/slow dissociation and high selectivity, and possesses strong in vivo and in vitro AChE-selective inhibitory activity, capable of improving learning and memory disorders. Studies have shown that 2-(4-fluoro-(1-(2-fluorobenzyl))piperidin-4-yl-methyl)-5,6-dimethoxy-2,3-dihydro-1-indanone has high oral bioavailability, good ability to cross the blood-brain barrier, and high drug concentration in brain tissue (4.4 times the plasma drug concentration); it has strong in vivo and in vitro AChE-selective inhibitory activity, good safety performance, and no hERG or genetic toxicity.

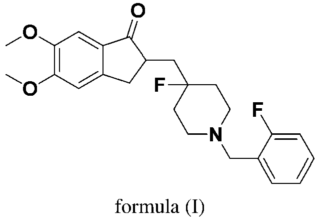

However, the compound of formula (I) or a pharmaceutically acceptable salt thereof is unstable under hot and humid conditions. Therefore, there is still a need to develop stable formulations that can provide the compound of formula (I) and can be produced on a large scale.

### SUMMARY OF THE INVENTION

To address the technical problems described above, the present application utilizes a process combining dry mixing and powder compression, thereby preparing a formulation of the compound of formula (I) with stable drug quality that can be produced on a large scale.

The present application provides a pharmaceutical composition comprising a compound of formula (I) or a pharmaceutically acceptable salt thereof, which has the advantages of high dissolution, good stability, low total impurity content, excellent content uniformity, and simple manufacturing process amenable to industrial production. The present application further provides a method for preparing the pharmaceutical composition.

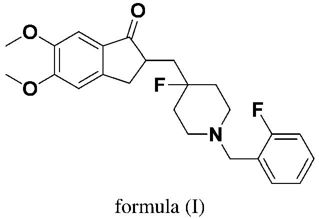

The chemical name of the compound of formula (I) is 2-(4-fluoro-(1-(2-fluorobenzyl))piperidin-4-yl-methyl)-5,6-dimethoxy-2,3-dihydro-1-indanone.

In one aspect, the present application provides a pharmaceutical composition comprising a compound of formula (I) or a pharmaceutically acceptable salt thereof, which further comprises one or more of a filler, a disintegrant, and a lubricant.

In some embodiments, in percent by weight, the pharmaceutical composition comprises 0.50 wt%-20.00 wt%, preferably 0.50 wt%-5.00 wt% of the compound represented by formula (I) or a pharmaceutically acceptable salt thereof. Preferably, the pharmaceutical composition comprises the compound of formula (I) or a pharmaceutically acceptable salt thereof having a particle size (D90) of 5 µm-50 µm, preferably 8 µm-12 µm.

In some embodiments, the filler is selected from pharmaceutical excipients with good compressibility. In some embodiments, the filler is selected from fillers comprising microcrystalline cellulose. In percent by weight, the filler accounts for 55.00 wt%-97.00 wt%, preferably 90.00 wt%-96.00 wt% of the weight of the pharmaceutical composition.

In some embodiments, in percent by weight, the disintegrant accounts for 0.50 wt%-7.00 wt%, preferably 2.00 wt%-5.50 wt% of the weight of the pharmaceutical composition.

In some embodiments, the lubricant is selected from one or more of colloidal silicon dioxide, sodium stearyl fumarate, and magnesium stearate. In percent by weight, the lubricant accounts for 0.10 wt%-5.00 wt%, preferably 0.20 wt%-2.00 wt% of the weight of the pharmaceutical composition.

In some embodiments, in percent by weight, the pharmaceutical composition comprises: 0.5 wt%-20 wt%, preferably 0.50 wt%-5.00 wt% of the compound of formula (I) or a pharmaceutically acceptable salt thereof; 55.00 wt%-97.00 wt%, preferably 90.00 wt%-96.00 wt% of a filler; 0.50 wt%-7.00 wt%, preferably 2.00 wt%-5.50 wt% of a disintegrant; and, 0.10 wt%-5.00 wt%, preferably 0.20 wt%-2.00 wt% of a lubricant. Preferably, in percent by weight, the pharmaceutical composition comprises: 0.50 wt%-4.00 wt% of the compound of formula (I) or a pharmaceutically acceptable salt thereof; 92.00 wt%-96.00 wt% of a filler; 2.00 wt%-5.50 wt% of a disintegrant; and, 0.20 wt%-2.00 wt% of a lubricant. Most preferably, in percent by weight, the pharmaceutical composition comprises: 0.50 wt%-2.50 wt% of the compound of formula (I) or a pharmaceutically acceptable salt thereof; 92.00 wt%-96.00 wt% of a filler; 2.00 wt%-5.50 wt% of a disintegrant; and, 0.20 wt%-2.00 wt% of a lubricant.

In a preferred embodiment, the pharmaceutical composition is in a dosage form for oral administration, such as a tablet.

In another aspect, the present application provides a method of preparing the pharmaceutical composition according to the present application, utilizing dry mixing, followed by powder compression.

In yet another aspect, the present application provides use of the pharmaceutical composition described above in the preparation of a medicament for treating nervous system disease associated with acetylcholinesterase, such as Alzheimer's disease, Parkinsonian syndrome, epilepsy, schizophrenia and the like.

In yet another aspect, the present application provides the pharmaceutical composition according to the present application for use in treating a nervous system disease associated with acetylcholinesterase. In some embodiments, the nervous system disease associated with acetylcholinesterase is, for example, Alzheimer's disease, Parkinsonian syndrome, epilepsy, schizophrenia, and the like.

In yet another aspect, the present application provides a method of treating a nervous system disease associated with acetylcholinesterase, comprising administering a therapeutically effective amount of the pharmaceutical composition according to the present application to a subject in need thereof. In some embodiments, the nervous system disease associated with acetylcholinesterase is, for example, Alzheimer's disease, Parkinsonian syndrome, epilepsy, schizophrenia, and the like.

Compared with the prior art, the present application provides the following advantages and excellent technical effects: the present application employs a combination of dry mixing and powder compression processes, with a simple and convenient preparation method, easy operation, and ready scalability for industrial production; and, the pharmaceutical composition of the present application has favorable stability, excellent content uniformity, and a slow increase in total impurities, which is advantageous for the production and storage of the product.

### EMBODIMENTS OF THE INVENTION

Exemplary embodiments of the present application will be described below, but the protection scope of the present application is not limited thereto.

### The pharmaceutical composition of the present application

The present application provides a pharmaceutical composition of the compound of formula (I) or a pharmaceutically acceptable salt thereof, which comprises the compound of formula (I) or a pharmaceutically acceptable salt thereof, and a pharmaceutically acceptable excipient.

In some embodiments, the pharmaceutically acceptable excipient is selected from one or more of a filler, a disintegrant, and a lubricant.

In some embodiments, the pharmaceutical composition comprises the compound of formula (I) or a pharmaceutically acceptable salt thereof, and one or more of a filler, a disintegrant, and a lubricant. The compound of formula (I), 2-(4-fluoro-(1-(2-fluorobenzyl))piperidin-4-yl-methyl)-5,6-dimethoxy-2,3-dihydro-1-indanone, is a structurally novel and highly selective small-molecule inhibitor of acetylcholinesterase (AChE), which is used for the treatment of nervous system diseases associated with acetylcholinesterase, such as Alzheimer's disease.

In some embodiments, the pharmaceutical composition is a pharmaceutical composition in a unit dose. In some embodiments, the amount of the compound of formula (I) or a pharmaceutically acceptable salt thereof in each unit dose of the composition is 0.1 mg-4.0 mg. In some embodiments, the amount of the compound of formula (I) in each unit dose of the composition is 0.1 mg, 0.2 mg, 0.3 mg, 0.4 mg, 0.5 mg, 0.6 mg, 0.7 mg, 0.8 mg, 0.9 mg, 1.0 mg, 1.1 mg, 1.2 mg, 1.3 mg, 1.4 mg, 1.5 mg, 1.6 mg, 1.7 mg, 1.8 mg, 1.9 mg, 2.0 mg, 2.1 mg, 2.2 mg, 2.3 mg, 2.4 mg, 2.5 mg, 2.6 mg, 2.7 mg, 2.8 mg, 2.9 mg, 3.0 mg, 3.1 mg, 3.2 mg, 3.3 mg, 3.4 mg, 3.5 mg, 3.6 mg, 3.7 mg, 3.8 mg, 3.9 mg, or 4.0 mg; or in a range formed by any of the foregoing values as endpoints. In some embodiments, the amount of the compound of formula (I) in each unit dose of the composition is selected from 0.1 mg-4.0 mg, 0.5 mg-4.0 mg, 1.0 mg-4.0 mg, and 1.0 mg-3.0 mg, for example, 1.0 mg, 2.0 mg, 3.0 mg, or 4.0 mg.

In some embodiments, the amount of the compound of formula (I) or a pharmaceutically acceptable salt thereof in the pharmaceutical composition according to the present application is selected from 0.50 wt%-20.00 wt%, preferably 0.50 wt%-5.00 wt%, and more preferably 0.50 wt%-2.50 wt%. In some embodiments, the amount of the compound of formula (I) or a pharmaceutically acceptable salt thereof in the pharmaceutical composition of the present application is selected from 0.50 wt%, 0.60 wt%, 0.70 wt%, 0.80 wt%, 0.90 wt%, 1.00 wt%, 1.50 wt%, 2.00 wt%, 2.50 wt%, 3.00 wt%, 3.50 wt%, 4.00 wt%, 4.50 wt%, 5.00 wt%, 5.50 wt%, 6.00 wt%, 6.50 wt%, 7.00 wt%, 7.50 wt%, 8.00 wt%, 8.50 wt%, 9.00 wt%, 9.50 wt%, 10.00 wt%, 10.50 wt%, 11.00 wt%, 11.50 wt%, 12.00 wt%, 12.50 wt%, 13.00 wt%, 13.50 wt%, 14.00 wt%, 14.50 wt%, 15.00 wt%, 15.50 wt%, 16.00 wt%, 16.50 wt%, 17.00 wt%, 17.50 wt%, 18.00 wt%, 18.50 wt%, 19.00 wt%, 19.50 wt%, or 20.00 wt%; or a range formed by any of the foregoing values as endpoints. In some embodiments, the amount of the compound of formula (I) in the pharmaceutical composition of the present application is selected from 0.50 wt%-5.00 wt%. In some embodiments, the amount of the compound of formula (I) in the pharmaceutical composition of the present application is selected form 0.50 wt%, 0.60 wt%, 0.70 wt%, 0.80 wt%, 0.90 wt%, 1.00 wt%, 1.10 wt%, 1.20 wt%, 1.30 wt%, 1.40 wt%, 1.50 wt%, 1.60 wt%, 1.70 wt%, 1.80 wt%, 1.90 wt%, 2.00 wt%, 2.10 wt%, 2.20 wt%, 2.30 wt%, 2.40 wt%, or 2.50 wt%, for example, 0.50 wt%-2.50 wt%.

In some embodiments, the filler is selected from pharmaceutical excipients with good compressibility. In some embodiments, the filler can be selected from one or more of microcrystalline cellulose and pregelatinized starch. In some embodiments, in percent by weight, the filler accounts for 55.00 wt%-97.00 wt%, preferably 90.00 wt%-96.00 wt% of the weight of the pharmaceutical composition.

In a preferred embodiment, at least 50% or more (such as 55% or more, 60% or more, or 65% or more) of the filler is a water-insoluble filler.

In some embodiments, the filler is microcrystalline cellulose, preferably in a range of 55.00 wt%-97.00 wt% of microcrystalline cellulose based on the total weight of the pharmaceutical composition.

In some embodiments, the filler is a filler comprising microcrystalline cellulose, preferably in a range of 55.00 wt%-80.00 wt% of microcrystalline cellulose, and preferably 60.00 wt%-80.00 wt% of microcrystalline cellulose based on the total weight of the pharmaceutical composition.

In some embodiments, the filler comprises pregelatinized starch, preferably in a range of 15.00 wt%-20.00 wt% of pregelatinized starch, and preferably 16.00 wt%-17.00 wt% of pregelatinized starch based on the total weight of the pharmaceutical composition.

In some embodiments, the filler is a combination of microcrystalline cellulose and pregelatinized starch. In a preferred embodiment, the mass ratio of microcrystalline cellulose to pregelatinized starch is from 5:1 to 1:1, such as from 5:1 to 1.5:1.

In some embodiments, the disintegrant is selected from one or more of starch and derivatives thereof (such as dry starch, sodium carboxymethyl starch), low-substituted hydroxypropylcellulose, cross-linked polyvinylpyrrolidone, croscarmellose sodium, and cross-linked povidone. In some embodiments, the disintegrant is one or more of low-substituted hydroxypropylcellulose; croscarmellose sodium; or cross-linked povidone and croscarmellose sodium. For example, the disintegrant is low-substituted hydroxypropylcellulose; cross-linked povidone; croscarmellose sodium; or low-substituted hydroxypropylcellulose, sodium carboxymethyl starch, and croscarmellose sodium.

In some embodiments, in percent by weight, the amount of the disintegrant in the pharmaceutical composition according to the present application is selected from 0.50 wt%-7.00 wt%, and preferably 2.00 wt%-5.50 wt%. In some embodiments, in the pharmaceutical composition of the present application, the amount of the disintegrant is selected from 0.50 wt%, 0.60 wt%, 0.70 wt%, 0.80 wt%, 0.90 wt%, 1.00 wt%, 1.10 wt%, 1.20 wt%, 1.30 wt%, 1.40 wt%, 1.50 wt%, 1.60 wt%, 1.70 wt%, 1.80 wt%, 1.90 wt%, 2.00 wt%, 2.10 wt%, 2.20 wt%, 2.30 wt%, 2.40 wt%, 2.50 wt%, 2.60 wt%, 2.70 wt%, 2.80 wt%, 2.90 wt%, 3.00 wt%, 3.10 wt%, 3.20 wt%, 3.30 wt%, 3.40 wt%, 3.50 wt%, 3.60 wt%, 3.70 wt%, 3.80 wt%, 3.90 wt%, 4.00 wt%, 4.10 wt%, 4.20 wt%, 4.30 wt%, 4.40 wt%, 4.50 wt%, 4.60 wt%, 4.70 wt%, 4.80 wt%, 4.90 wt%, 5.00 wt%, 5.50 wt%, 6.00 wt%, 6.50 wt%, or 7.00 wt%; or in a range formed by any of the foregoing values as endpoints. In some embodiments, in the pharmaceutical composition of the present application, the amount of the disintegrant is selected from 2.00 wt%-4.50 wt%.

In one embodiment, the disintegrant is low-substituted hydroxypropylcellulose and/or croscarmellose sodium. In one embodiment, the disintegrant is low-substituted hydroxypropylcellulose. In one embodiment, the disintegrant is croscarmellose sodium. In one embodiment, the disintegrant is a combination of low-substituted hydroxypropylcellulose and croscarmellose sodium.

In the case where the disintegrant is low-substituted hydroxypropylcellulose and croscarmellose sodium, the two can be combined in any ratio. For example, the mass ratio of low-substituted hydroxypropylcellulose and croscarmellose sodium can be from 10:1 to 1:10, such as 10:1, 5:1, 1:1, 1:2, 1:3, 1:5, or 1:10.

In some embodiments, the lubricant is selected from one or more of colloidal silicon dioxide, sodium stearyl fumarate, and magnesium stearate. In some embodiments, the lubricant is selected from magnesium stearate; sodium stearyl fumarate; and, magnesium stearate and colloidal silicon dioxide.

In some embodiments, in percent by weight, the amount of the lubricant in the pharmaceutical composition according to the present application is selected from 0.10 wt%-5.00 wt%, and preferably 0.20 wt%-2.00 wt%. In some embodiments, in the pharmaceutical composition of the present application, the amount of the lubricant is selected from 0.10 wt%, 0.20 wt%, 0.30 wt%, 0.40 wt%, 0.50 wt%, 0.60 wt%, 0.70 wt%, 0.80 wt%, 0.90 wt%, 1.00 wt%, 1.10 wt%, 1.20 wt%, 1.30 wt%, 1.40 wt%, 1.50 wt%, 1.60 wt%, 1.70 wt%, 1.80 wt%, 1.90 wt%, 2.00 wt%, 2.10wt%, 2.20wt%, 2.30wt%, 2.40wt%, 2.50wt%, 2.60wt%, 2.70wt%, 2.80wt%, 2.90wt%, 3.00wt%, 3.10 wt%, 3.20 wt%, 3.30 wt%, 3.40 wt%, 3.50 wt%, 3.60 wt%, 3.70 wt%, 3.80 wt%, 3.90 wt%, 4.00 wt%, 4.10 wt%, 4.20 wt%, 4.30 wt%, 4.40 wt%, 4.50 wt%, 4.60 wt%, 4.70 wt%, 4.80 wt%, 4.90 wt%, or 5.00 wt%; or a range formed by any of the foregoing values as endpoints.

In the case where the lubricant is a combination of magnesium stearate and colloidal silicon dioxide, the mass ratio of magnesium stearate to colloidal silicon dioxide can be 1:1 or greater, such as, 1:1, 2:1, 3:1, 4:1, 5:1, 6:1, or 10:1. The particle size D90 as described in the present application refers to the particle size at which the cumulative particle size distribution of a sample reaches 90%, and its physical significance is that 90% of the particles have a particle size smaller than it.

In some embodiments of the present application, the pharmaceutical composition comprises the compound of formula (I) or a pharmaceutically acceptable salt thereof having a particle size (D90) of 5 µm-50 µm, and preferably 8 µm-12 µm.

In some embodiments, the dosage form of the pharmaceutical composition can be selected from tablets, capsules, powders, pills, pellets, pastes, dispersions, or inhalable powders, preferably tablets.

In some embodiments, the pharmaceutical composition is formulated as a pharmaceutical composition in a unit dose form. In some embodiments, the pharmaceutical composition in a unit dose form comprises 0.5 mg-4 mg of the compound of formula (I) or a pharmaceutically acceptable salt thereof. In some embodiments, the pharmaceutical composition in a unit dose form comprises 0.5 mg-4.0 mg of the compound of formula (I) or a pharmaceutically acceptable salt thereof. In some embodiments, the pharmaceutical composition in a unit dose form comprises 0.5 mg-3.0 mg of the compound of formula (I) or a pharmaceutically acceptable salt thereof. In some particular embodiments, the pharmaceutical composition in a unit dose form comprises 0.5 mg, 1.0 mg, 1.48 mg, 1.65 mg, 1.7 mg, 2 mg, 2.1 mg, 2.3 mg, 3.0 mg, or 4.0 mg of the compound of formula (I) or a pharmaceutically acceptable salt thereof.

In some particular embodiments, the pharmaceutical composition in a unit dose form is a tablet containing 1 mg of the compound represented by formula (I) or a pharmaceutically acceptable salt thereof. In some particular embodiments, the pharmaceutical composition in a unit dose form is a tablet containing 2 mg of the compound represented by formula (I) or a pharmaceutically acceptable salt thereof. In some particular embodiments, the pharmaceutical composition in a unit dose form is a tablet containing 3 mg of the compound represented by formula (I) or a pharmaceutically acceptable salt thereof. In some particular embodiments, the pharmaceutical composition in a unit dose form is a tablet containing 4 mg of the compound represented by formula (I) or a pharmaceutically acceptable salt thereof.

The skilled in the art will also understand that, in addition to the active ingredient compound of formula (I) and the filler, disintegrant, or lubricant as described above, the pharmaceutical composition of the present application can further comprise other pharmaceutically acceptable excipients or auxiliaries as needed, such as but not limited to diluents, antioxidants, preservatives, coloring agents, flavoring agents, coating agents, and the like, the selection and amount of which can be adjusted by the skilled in the art according to actual needs. In a preferred embodiment, the coating agent can be selected from a gastric-soluble coating agent or an enteric coating agent.

The functions of pharmaceutical excipients are not singular, and many excipients serve multiple purposes. Due to the poor stability and low dose of the compound of formula (I), the selection of diluents differs accordingly. Typically, in the pharmaceutical field, diluents are also categorized as fillers. However, the diluents used in the present application are distinguished from conventional fillers. Considering the improvement of drug flowability and content uniformity of the finished formulation, lactose and mannitol are not preferred as diluents in the present application because their inherent hygroscopicity and hydrophilicity result in relatively poor performance. Previous studies on the compound of formula (I) were limited to animal experiments only, and the compositions used therein failed to meet the requirements for pharmaceutical manufacturing and clinical trials. Additionally, the stability of compositions of the compound of Formula (I) had not been thoroughly investigated. The compound of formula (I) exhibits instability under hot and humid conditions. The pharmaceutical composition according to the present application substantially improves quality indicators of the finished product (such as tablets), such as content uniformity, related substances, dissolution profile, thereby effectively ensuring drug quality. Furthermore, through standardized clinical trials, it has been established that the pharmaceutical composition of the present application can satisfy the requirements for large-scale manufacturing, clinical research at different stages, and industrial preparation processes. In addition, the quality reliability and stability of the pharmaceutical composition of the present application have been confirmed through comprehensive standardized pharmaceutical research.

### Embodiments of the pharmaceutical composition

In some embodiments of the present application, relative to the total weight of the pharmaceutical composition, the pharmaceutical composition of the present application comprises: 0.50 wt%-20.00 wt%, preferably 0.50 wt%-5.00 wt% of the compound of formula (I) or a pharmaceutically acceptable salt thereof; 55.00 wt%-97.00 wt%, preferably 90.00 wt%-96.00 wt% of a filler, wherein the filler is selected from pharmaceutical excipients with good compressibility; 0.50 wt%-7.00 wt%, preferably 2.00 wt%-5.50 wt% of a disintegrant, wherein the disintegrant is selected from one or more of starch and derivatives thereof (such as dry starch, sodium carboxymethyl starch), low-substituted hydroxypropylcellulose, cross-linked polyvinylpyrrolidone, croscarmellose sodium, and cross-linked povidone; and, 0.10 wt%-5.00 wt%, preferably 0.20 wt%-2.00 wt% of a lubricant, wherein the lubricant is selected from one or more of colloidal silicon dioxide, sodium stearyl fumarate, and magnesium stearate.

In some embodiments of the present application, the pharmaceutical composition of the present application comprises: 0.50 wt%-4.00 wt% of the compound of formula (I) or a pharmaceutically acceptable salt thereof; 65 wt%-97.00 wt% of a filler, wherein the filler is microcrystalline cellulose; 0.50 wt%-7.00 wt% of a disintegrant, wherein the disintegrant is selected from one or more of low-substituted hydroxypropylcellulose and croscarmellose sodium; and, 0.10 wt%-5.00 wt% of a lubricant, wherein the lubricant is selected from one or more of colloidal silicon dioxide, sodium stearyl fumarate, and magnesium stearate. In some embodiments of the present application, the pharmaceutical composition of the present application comprises: the compound of formula (I) or a pharmaceutically acceptable salt thereof; microcrystalline cellulose; one or more of low-substituted hydroxypropylcellulose and/or croscarmellose sodium; and one or more of colloidal silicon dioxide, sodium stearyl fumarate, and magnesium stearate.

In some embodiments of the present application, relative to the total weight of the pharmaceutical composition, the pharmaceutical composition of the present application comprises: 0.50 wt%-4.00 wt% of the compound of formula (I) or a pharmaceutically acceptable salt thereof; 65 wt%-97 wt% of a filler, wherein the filler is a combination of microcrystalline cellulose and pregelatinized starch; 0.50 wt%-7.00 wt% of a disintegrant, wherein the disintegrant is low-substituted hydroxypropylcellulose; and, 0.10 wt%-5.00 wt% of a lubricant, wherein the lubricant is sodium stearyl fumarate or magnesium stearate.

In some embodiments of the present application, relative to the total weight of the pharmaceutical composition, the pharmaceutical composition of the present application comprises: 0.50 wt%-4.00 wt% of the compound of formula (I) or a pharmaceutically acceptable salt thereof; 65 wt%-97 wt% of a filler, wherein the filler is a combination of microcrystalline cellulose and pregelatinized starch; 0.50 wt%-7.00 wt% of a disintegrant, wherein the disintegrant is low-substituted hydroxypropylcellulose; and, 0.10 wt%-5.00 wt% of a lubricant, wherein the lubricant is a combination of colloidal silicon dioxide and magnesium stearate.

In some embodiments of the present application, relative to the total weight of the pharmaceutical composition, the pharmaceutical composition of the present application comprises: 0.50 wt%-4.00 wt% of the compound of formula (I) or a pharmaceutically acceptable salt thereof; 65 wt%-97 wt% of a filler, wherein the filler is a combination of microcrystalline cellulose and pregelatinized starch; 0.50 wt%-7.00 wt% of a disintegrant, wherein the disintegrant is low-substituted hydroxypropylcellulose; and, 0.10 wt%-5.00 wt% of a lubricant, wherein the lubricant is magnesium stearate.

In some embodiments of the present application, relative to the total weight of the pharmaceutical composition, the pharmaceutical composition of the present application comprises:
0.50 wt%-2.50 wt% of the compound of formula (I) or a pharmaceutically acceptable salt thereof; 92.00 wt%-96.00 wt% of a filler, wherein the filler is a combination of microcrystalline cellulose and pregelatinized starch; 2.00 wt%-4.50 wt% of a disintegrant, wherein the disintegrant is croscarmellose sodium; and, 0.20 wt%-2.00 wt% of a lubricant, wherein the lubricant is magnesium stearate.

In some embodiments, the pharmaceutical composition comprises the compound of formula (I) or a pharmaceutically acceptable salt thereof having a particle size (D90) of 5 µm-50 µm, preferably 8 µm-12 µm.

In some embodiments of the present application, the pharmaceutical composition of the present application is in the form of a tablet. And, the thickness of the tablet is 1 mm-10 mm, preferably 3 mm-5 mm.

### Preparation method

In another aspect, the present application provides a method of preparing the pharmaceutical composition according to the present application.

In one embodiment, the present application provides a method of preparing a pharmaceutical composition in the form of a tablet.

In some embodiments, the pharmaceutical composition of the present application is prepared using a method comprising dry mixing, followed by powder compression.

In some embodiments, the compound of formula (I) or a pharmaceutically acceptable salt thereof is mixed together simultaneously with the filler, the disintegrant, and the lubricant. Alternatively, in some embodiments, the compound of formula (I) is mixed sequentially with the filler, the disintegrant, and the lubricant. For example, the compound of formula (I) or a pharmaceutically acceptable salt thereof can be mixed first with the filler, subsequently with the disintegrant, and then with the lubricant.

In some embodiments, the active pharmaceutical ingredient used in the present application (the compound represented by formula (I) or a pharmaceutically acceptable salt thereof) is micronized to provide an active pharmaceutical ingredient having a particle size (D90) of 5 µm-50 µm, preferably 8 µm-12 µm. In some embodiments, the active pharmaceutical ingredient used in the present application is micronized by jet milling and then sieved, preferably using a sieve of 80 mesh-120 mesh.

In some embodiments, the dissolution rate of the drug is significantly improved by mixing the micronized active pharmaceutical ingredient (the compound of Formula (I) or a pharmaceutically acceptable salt thereof) used in the present application with excipients (excluding the lubricant) by geometric dilution. In some embodiments, the active pharmaceutical ingredient used in the present application is micronized and mixed with the filler and the disintegrant by geometric dilution, and the resulting mixture is then mixed with the lubricant; alternatively, the active pharmaceutical ingredient used in the present application is micronized and mixed with the filler by geometric dilution, and the resulting mixture is then mixed with the disintegrant and the lubricant simultaneously or sequentially; alternatively, the active pharmaceutical ingredient used in the present application is micronized and mixed with a portion of the filler by geometric dilution, and the resulting mixture is then mixed with the remaining filler, the disintegrant, and the lubricant simultaneously or sequentially.

In the present application, "geometric dilution" means mixing the active pharmaceutical ingredient with an equal amount or a multiple amount (preferably 2-fold, 3-fold, or 4-fold) of filler, then adding an amount of filler equal to the total weight of the previous mixing, and continuing in this manner until all filler is incorporated. Preferably, the active pharmaceutical ingredient is mixed with filler in an equal amount or a 2-fold amount, such as the active pharmaceutical ingredient + a 2-fold amount of filler. For example, in some embodiments, mixing is performed by sieving, or using a mixer during geometric dilution. For example, the filler in a double amount relative to the active pharmaceutical ingredient is first mixed with the active pharmaceutical ingredient, and the same multiple is used for the next two mixing. For example, 2 parts by weight of the active pharmaceutical ingredient and 4 parts by weight of a filler (e.g., microcrystalline cellulose + pregelatinized starch), which is double the amount of the active pharmaceutical ingredient, are mixed and sieved for the first time;then 6 parts by weight of the filler are added to the first mixture and mixed and sieved for the second time; and so on until all of the components are completely added.

In some embodiments, when mixing is perfomed using a mixer, the following parameters can be employed for mixing with the filler and the disintegrant: a mixing speed of 20 rpm-40 rpm, preferably 25 rpm-35 rpm, preferably 30 rpm; and a mixing time of 20 min-25 min. The mixing parameters as described above can also be employed for mixing during the geometric dilution.

In some embodiments, when mixing is performed using a mixer, the following parameters can be employed for mixing with the lubricant: a mixing speed of 20 rpm-40 rpm, preferably 25 rpm-35 rpm, preferably 30 rpm; and a mixing time of 5 min-10 min.

In the mixing operation of the method, the equipment used is a three-dimensional motion mixer.

In the method of the present application, compression is performed using a rotary tablet press in a conventional manner.

In a preferred embodiment, the tablet obtained has a thickness of 1 mm-10 mm, preferably 3 mm-5 mm.

In some embodiments, the method comprises:
Micronization: micronizing the active pharmaceutical ingredient to give the active pharmaceutical ingredient having a particle size (D90) of 5 µm-50 µm, preferably 8 µm-12 µm;
Mixing by geometric dilution: mixing the micronized active pharmaceutical ingredient with at least a portion of a filler by geometric dilution;
Final blending: mixing the mixture obtained by mixing by geometric dilution, with the remaining portion of the filler, a disintegrant, and a lubricant simultaneously or sequentially;
Compressing: compressing the final blended mixture.

In some embodiments, the method of preparing a pharmaceutical composition of the compound of formula (I) or a pharmaceutically acceptable salt thereof according to the present application comprises:
micronizing the active pharmaceutical ingredient to provide Material 1 with a suitable particle size;
Mixing Material 1 with at least a portion of a filler by geometric dilution to provide Mixture 1, wherein each mixing is performed at 20 rpm-40 rpm for 20 min-25 min;
Mixing Mixture 1, with the remaining portion of the filler and a disintegrant at 20 rpm-40 rpm for 20 min-25 min, to provide Mixture 2;
Adding a lubricant to Mixture 2 and mixing at 20 rpm-40 rpm for 5 min-10 min, to provide the final blended mixture; compressing the final blended mixture using a die, to form tablets having a thickness of 1 mm-10 mm, preferably 3 mm-5 mm.

In some embodiments, the method of preparing a pharmaceutical composition of the compound of formula (I) or a pharmaceutically acceptable salt thereof according to the present application comprises:
1) micronizing the active pharmaceutical ingredient to provide Material 1 with a D90 of 8 µm-12 µm;
2) mixing Material 1 with one of the fillers by geometric dilution to provide Mixture 1, wherein each mixing is performed at 20 rpm-40 rpm for 20 min-25 min;
3) mixing Mixture 1, with the remaining portion of the fillers and a disintegrant at 20 rpm-40 rpm for 20 min-25 min, to provide Mixture 2;
4) adding a lubricant to Mixture 2 and mixing at 20 rpm-40 rpm for 5 min-10 min to provide the final blended mixture;
5) compressing the final blended mixture using a die, to form tablets having a thickness of 3 mm-5 mm.

In a specific embodiment, the method of preparing a pharmaceutical composition of the compound of formula (I) or a pharmaceutically acceptable salt thereof according to the present application comprises:
1) micronizing the active pharmaceutical ingredient to provide Material 1 with a D90 of 8 µm to 12 µm;
2) mixing Material 1 and pregelatinized starch by geometric dilution to provide Mixture 1, wherein Material ① and pregelatinized starch are first mixed in a ratio of 1:2, followed by mixing by geometric dilution, wherein each mixing is performed at 20 rpm-40 rpm for 20 min-25 min;
3) mixing Mixture 1 with microcrystalline cellulose and low-substituted hydroxypropylcellulose at 20 rpm-40 rpm for 20 min-25 min to provide Mixture 2;
4) adding magnesium stearate to Mixture 2 and mixing at 20 rpm-40 rpm for 5 min-10 min to provide the final blended mixture;
5) compressing the final blended mixture using a die, to form tablets having a thickness of 3 mm-5 mm.

The selection and dosage of the compound of formula (I) or a pharmaceutically acceptable salt thereof, as well as the filler, disintegrant, and lubricant used in the method are as described above.

In some embodiments, the pharmaceutical composition is a solid pharmaceutical composition. In some embodiments, the solid pharmaceutical composition can further comprise a coating agent. In some embodiments, a coating agent is formed from an aqueous film coating composition, wherein the aqueous film coating composition comprises a film-forming polymer, water and/or alcohol as a carrier, and optionally one or more auxiliaries, such as those known in the field of film coating. In some embodiments, the coating agent is selected from the group consisting of hydroxypropylmethylcellulose, hydroxypropylcellulose, titanium dioxide, talc, polyethylene glycol, propylene glycol, castor oil, diethyl phthalate, glyceryl monostearate, and the like.

In the present application, the compound of formula (I) is micronized (e.g., by jet milling) in combination with specific excipient constitution and powder compression, allowing content uniformity, related substances, dissolution rate, and other quality indicators of the intermediates and finished products prepared through dry mixing to be greatly improved. This thereby ensures the effective quality of the drug and maximally meeting the on-site production process requirements for large-scale manufacturing and clinical sample preparation.

### Method and use for treatment or prevention

The present application provides use of the pharmaceutical composition as described above in the preparation of a medicament for treating a nervous system disease associated with acetylcholinesterase, such as Alzheimer's disease, Parkinsonian syndrome, epilepsy, schizophrenia and the like.

Another aspect of the present application provides a method of treating a nervous system disease associated with acetylcholinesterase, such as Alzheimer's disease, Parkinsonian syndrome, epilepsy, schizophrenia, and the like, comprising administering a therapeutically effective amount of the pharmaceutical composition according to the present application to a subject in need thereof.

The present application provides the pharmaceutical composition according to the present application for use in treating a nervous system disease associated with acetylcholinesterase-. In some embodiments, the nervous system disease associated with acetylcholinesterase is, for example, Alzheimer's disease, Parkinsonian syndrome, epilepsy, schizophrenia, and the like.

### ADVANTAGEOUS EFFECTS

The present application provides the following advantages and excellent technical effects over existing technologies, by utilizing a distinctive geometric dilution approach combined with dry mixing and powder compression processes tailored to the drug's physicochemical properties and stability data: it effectively improves drug dispersion uniformity, preserves the chemical composition and structural integrity of the active pharmaceutical ingredient, and enhances steady-state absorption and distribution *in vivo;* and the efficacy of the drug has been validated through rigorous clinical pharmacodynamic and pharmacokinetic studies, establishing broad prospects for clinical application via oral administration in humans. The method of preparing the pharmaceutical composition of the present application is simple and convenient to operate, and can be applied to batch preparation of clinical samples and large-scale production; the resulting tablets exhibit significantly improved dissolution profiles under identical conditions, solve the problem of failed content uniformity during batch production, and demonstrate stable related substances, ensuring effective quality control.

Unless otherwise indicated, for the purposes of the present application, the following terms used in the present specification and claims shall have the following meanings.

Unless otherwise defined, technical and scientific terms used herein have the same meaning as commonly understood by those skilled in the art to which the present disclosure belongs.

The term "comprise" and English variants thereof, such as "comprises" or "comprising", are intended as an open and non-exclusive sense, that is, "including but not limited to/including without limitation".

Unless otherwise specifically defined, any ratios (including percentages) or parts used herein are by weight.

The term "pharmaceutically acceptable" refers to those compounds, materials, compositions and/or dosage forms that are, within the scope of sound medical judgment, suitable for use in contact with the tissues of humans and animals without excessive toxicity, irritation, allergic response or other problems or complications, commensurate with a reasonable benefit-to-risk ratio.

The term "pharmaceutical composition" refers to a mixture comprising one or more active ingredients of the present disclosure and pharmaceutically acceptable excipients. The pharmaceutical composition is intended to facilitate the administration of the compound of the present disclosure to a subject.

Herein, when referring to the compound of formula (I) or a pharmaceutically acceptable salt thereof herein, the amount is calculated as the compound represented by formula (I).

The compound of formula (I) can be administered in the form of its free base, or in the form of a salt, solvate, or prodrug, where the prodrug is converted in vivo into the free base form of the compound of formula (I). For example, pharmaceutically acceptable salts of the compound of formula (I) fall within the scope of the present application, and may be produced from various organic acids and inorganic acids according to methods well known in the art.

In some embodiments, the pharmaceutically acceptable salt of the compound of formula (I) of the present application refers to a salt formed by the compound of formula (I) and a pharmaceutically acceptable acid, wherein the pharmaceutically acceptable acid is selected from sulfuric acid, carbonic acid, nitric acid, hydrochloric acid, hydrobromic acid, hydroiodic acid, phosphoric acid, metaphosphoric acid, trifluoroacetic acid, lactic acid, mandelic acid, glycolic acid, *p*-toluenesulfonic acid, *o*-toluenesulfonic acid, citric acid, methanesulfonic acid, formic acid, acetic acid, benzoic acid, phenylacetic acid, malonic acid, cinnamic acid, malic acid, maleic acid, tartaric acid, oxalic acid, fumaric acid, acrylic acid, crotonic acid, oleic acid and linoleic acid. In some embodiments, the pharmaceutically acceptable acid is selected from sulfuric acid, hydrochloric acid, phosphoric acid, *p-*toluenesulfonic acid, citric acid, methanesulfonic acid, malic acid, maleic acid, tartaric acid, and fumaric acid. In some embodiments, the molar ratio of the compound of formula (I) to the pharmaceutically acceptable acid in the salt is 1:0.5, 1:1, 1:1.5, 1:2, 1:2.5, 1:3, 1:3.5, or 1:4.

The term "administration" or "administer" or "administering" means physically introducing a composition comprising an active compound into a individual or patient or subject using any of the various methods and delivery systems known to the skilled in the art. In certain embodiments, the administration is oral.

The term "unit dosage" or "unit dose" refers to a pharmaceutical composition packaged in a single package for convenience of administration, for example, "a pharmaceutical composition in a unit dose form" means each tablet. "A pharmaceutical composition in a unit dose form containing 2 mg of the compound of formula (I)" means that each tablet finally prepared contains 2 mg of the compound of formula (I).

The term "patient", "subject", or "individual" refers to a mammal, preferably a human.

The term "therapeutically effective amount" means an amount of a compound that, when administered to a human for treating a disease, is sufficient to achieve treatment of the disease.

The term "treat", "treating" or "treatment" means any administration of a therapeutically effective amount of a compound, and includes:
(1) inhibiting the disease in a human being who is experiencing or manifesting the pathology or symptomology of the disease (i.e., arresting the further development of the pathology and/or symptomology), or
(2) ameliorating the disease in a human who is experiencing or manifesting the pathology or symptomology of the disease (i.e., reversing the pathology and/or symptomology).

All patents, patent applications, and other publications identified are hereby expressly incorporated herein by reference herein for purposes of description and disclosure. Any reference to these publications herein does not constitute an admission that such publication forms part of the common general knowledge in the art.

### Example

The examples described hereinafter are provided by way of illustration only of the content of the present invention, but do not constitute any limitation on the scope of the present application. The excipients and reagents used are commercially available products. The instruments and equipment used are those commonly used in the field.

### Analysis of active compounds:

Solvent: acetonitrile-water = 50:50; Mobile phase: Mobile Phase A: phosphate solution (2.72 g of potassium dihydrogen phosphate and 3 mL of triethylamine were taken, dissolved in water, and diluted to 1000 mL, and the pH value was then adjusted to 6.0 with phosphoric acid)-acetonitrile (90:10); Mobile Phase B: acetonitrile; A:B = 61:39(%).

Reference Standard Solution: 10 mg of the compound of formula (I) (reference substance) was accurately weighed, placed into a 100-mL volumetric flask, and dissolved in the solvent to volume, to give a reference standard solution containing 100 µg/mL of the compound of formula (I).

Test Solution: 20 tablets of the product were accurately taken, and ground finely. A portion of the powder (equivalent to about 10 mg of the compound of formula (I)) was accurately weighed, placed in a 100-mL volumetric flask, added with an appropriate amount of the solvent to dissolve the compound of formula (I) by sonication, then diluted to volume with the solvent, mixed, and filtered. The subsequent filtrate was accurately measured for use as the test solution.

### Chromatographic conditions

Detection wavelength: UV270 nm; chromatography column: 4.6 mm×15 cm; 3.5 µm, C18 column, model: Waters Xbridge C18; column temperature: 35°C; flow rate: 1.0 mL/min; injection volume: 10 µL; run time: 20 min. Determination of Total impurities: HPLC method (Chinese Pharmacopoeia (2015 Edition), Part IV, General Chapter 0512).

Wherein, solvent: acetonitrile-water = 50:50; Mobile phase: Mobile Phase A: phosphate solution (2.72 g of potassium dihydrogen phosphate and 3 mL of triethylamine were taken, dissolved in water, and diluted to 1000 mL, and the pH value was adjusted to 6.0 with phosphoric acid)-acetonitrile (80:20); Mobile Phase B: acetonitrile; Mobile phase gradient table

| Time (min) | Mobile Phase A (%) | Mobile Phase B(%) |
|---|---|---|
| 0 | 100 | 0 |
| 20 | 75 | 25 |
| 40 | 63 | 37 |
| 45 | 38 | 62 |
| 50 | 38 | 62 |

Test Solution: 20 tablets of the product were accurately taken, and ground finely. A portion of the powder (equivalent to about 10 mg of the compound of formula (I)) was accurately weighed, placed into a 20-mL volumetric flask, added with an appropriate amount of the solvent to dissolve the compound of formula (I) by sonication, then diluted to volume with the solvent, mixed, and filtered, to provide the test solution.

Reference Standard Solution: 10 mg of the compound of formula (I) (reference substance) was accurately weighed and placed into a 20-mL volumetric flask, dissolved in the solvent and diluted to volume. Then, 1 mL of the solution was placed into a 100-mL volumetric flask, diluted to volume with solvent, and mixed well, to provide the reference standard solution.

### Chromatographic conditions

Detection wavelength: UV270 nm; chromatography column: 4.6 mm×15 cm; 3.5 µm, C18 column, model: Waters Xbridge C18; column temperature: 35°C; flow rate: 1.0 mL/min; injection volume: 10 µL; run time: 50 min.

Dissolution testing: As for the tablets, the dissolution test was perfomed by the paddle method according to Method II (for dissolution) of General Chapter of the Chinese Pharmacopoeia (2015 Edition, Part II). Wherein, the dissolution medium was a pH 4.5 acetate buffer (prepared by dissolving 2.99 g of sodium acetate and 14.0 mL of 2 mol/L acetic acid solution in water, diluting to 1000 mL, and mixing well), 500 mL.

Hardness determination: Hardness was measured using a YPD-300D intelligent tablet hardness tester from Shanghai Huanghai Drug Testing Instrument Co., Ltd. A hardness range of 2-12 kg was considered desirable for the tablets. Friability determination: Friability was determined using an FT-2000A friabilator from Radio Factory Tianjin University, in accordance with the test method for friability of tablets (Chinese Pharmacopoeia 2015 Edition, Part IV, General Chapter 0923).

Microbial limit determination: Microbial limits were determined with reference to the test for microbial limits (Chinese Pharmacopoeia 2015 Edition, Part IV General Chapters 1105 and 1106 in).

Content uniformity determination: Chinese Pharmacopoeia 2015 Edition, Part IV, General Chapter 0941, using the HPLC method (Chinese Pharmacopoeia 2015 Edition, Part IV, General Chapter 0512).

### Preparation Example 1: Preparation of the compound of formula (I)

The compound of formula (I) was prepared according to the method disclosed in CN 103787954 A.

### Preparation Example 2: Study on the dosage form of the compound of formula (I)

### 2.1 Selection of filler type and proportion of

Commonly used fillers for tablets, such as mannitol, microcrystalline cellulose, and pregelatinized starch, were chosen. The influence of filler type and proportion on the tablets of the compound of formula (I) was subsequently investigated, with the appearance, hardness, friability, and dissolution of the finished tablets serving as screening parameters.

**Table 1: Selection of filler types and proportions and the results**

| Component | Formulation 1 | Formulation 2 | Formulation 3 | Formulation 4 | Formulation 5 |
|---|---|---|---|---|---|
| Compound of formula (I) | 2 mg | 2 mg | 2 mg | 2 mg | 2 mg |
| Microcrystalline cellulose | 122 mg | / | / | 100 mg | 80 mg |
| Pregelatinized starch | / | 122 mg | / | 22 mg | 42 mg |
| Mannitol | / | / | 122 mg | / | / |
| Low-substituted hydroxypropylcellulose | 5 mg | 5 mg | 5 mg | 5 mg | 5 mg |
| Magnesium stearate | 1 mg | 1 mg | 1 mg | 1 mg | 1 mg |

| Test Items | | | | | |
|---|---|---|---|---|---|
| Appearance | Pass | Chipping | Sticking | Pass | Pass |
| Hardness | 7.90 kg | 2.19 kg | 3.28 kg | 7.33 kg | 6.52 kg |
| Friability | 0.12% | 2.36% | 1.31% | 0.15% | 0.55% |

The compound of formula (I) in the proportions set forth in the formulations and the excipients listed in Table 1 were mixed homogeneously and compressed into tablets. The relevant parameters of the tablets were then tested. Among them, Formulations 2 and 3 were not further studied due to issues such as appearance defects, poor hardness, friability failure. Formulations 1, 4, and 5 were selected for further dissolution evaluation. The tablets were subjected to dissolution testing at 37°C and 50 rpm using 500 mL of the pH 4.5 acetate buffer as the dissolution medium, with sampling at 5, 10, 15, 30, 45, and 60 minutes for determination of drug dissolution.

**Table 2 Dissolution test results of Formulations 1, 4, and 5**

| Time (min) | Dissolution rate (%) | | |
|---|---|---|---|
| | Formulation 1 | Formulation 4 | Formulation 5 |
| 5 | 11.3 | 48.7 | 52.9 |
| 10 | 20.2 | 69.7 | 71.1 |
| 15 | 27.9 | 84.5 | 78.6 |
| 30 | 44.4 | 91.6 | 87.4 |
| 45 | 56.0 | 97.4 | 90.7 |
| 60 | 63.8 | 101.0 | 93.4 |

As can be seen from the dissolution results in Table 2 above, the compound of formula (I) in Formulation 1 failed to completely dissolve in the medium at pH 4.5, whereas Formulations 4 and 5 demonstrated favorable dissolution profiles.

Based on the screening described above, it was found that when mannitol and pregelatinized starch were used alone as fillers, the prepared tablets exhibited phenomena such as sticking or cracking, and had poor hardness and non-compliant friability; when microcrystalline cellulose was used alone as a filler, the tablets failed to dissolve completely. In view of the high cost associated with direct-compression grade mannitol and its compatibility concerns under high-humidity conditions, the combination of pregelatinized starch and microcrystalline cellulose was preferred. While the inclusion of pregelatinized starch could accelerate the disintegration and dissolution rate of the tablets, it may concurrently reduce the hardness of the tablets, and adversely affect friability of the drug. Therefore, further studies were conducted based on the filler proportions in Formulation 4.

### 2.2 Selection of disintegrant type and proportion

Commonly used disintegrants for tablets, such as low-substituted hydroxypropylcellulose and croscarmellose sodium, were chosen. The influence of disintegrant type and proportion on the tablets of the compound of formula (I) was subsequently investigated, with the appearance, disintegration time, hardness, friability, and dissolution of the finished tablets serving as screening parameters. Wherein, microcrystalline cellulose and pregelatinized starch were used as the fillers, and the specific formulations are shown in Table 3 below.

**Table 3: Selection of disintegrant types and proportions and the results**

| Component | Formulation 4 | Formulation 6 | Formulation 7 | Formulation 8 |
|---|---|---|---|---|
| Compound of formula (I) | 2 mg | 2 mg | 2 mg | 2 mg |
| Microcrystalline cellulose | 100 mg | 100 mg | 100 mg | 100 mg |
| Pregelatinized starch | 22 mg | 22 mg | 22 mg | 22 mg |
| Low-substituted hydroxypropylcellulose | 5 mg | / | 3 mg | 7 mg |
| Croscarmellose sodium | / | 5 mg | / | / |
| Magnesium stearate | 1 mg | 1 mg | 1 mg | 1 mg |

| Test Items | | | | |
|---|---|---|---|---|
| Appearance | Pass | Pass | Pass | Pass |
| Disintegration Time | 3min | 3min | 5min | 2min |
| Hardness | 7.33 kg | 5.98 kg | 6.52 kg | 7.12 kg |
| Friability | 0.14% | 0.17% | 0.15% | 0.09% |

The compound of formula (I) in the proportions set forth in the formulations and the excipients in the proportions specified in Table 3 were mixed homogeneously and compressed into tablets. The relevant parameters of the tablets were then tested. Among them, based on the comparison of disintegration time, it was revealed that the various disintegrants exhibited substantially equivalent disintegration performance. Increased disintegrant proportions can result in accelerated tablet disintegration. Tablet hardness was additionally determined. Comparative analysis of hardness and flowability results indicated that low-substituted hydroxypropyl cellulose was more conducive to attaining higher hardness. The prepared tablets were subjected to dissolution testing at 37 °C and 50 rpm using 500 mL of the pH 4.5 acetate buffer as the dissolution medium, with sampling at 5, 10, 15, 30, 45, and 60 minutes for determination of drug dissolution.

**Table 4: Dissolution test results of Formulations 4, 7, and 8**

| Time (min) | Dissolution rate (%) | | |
|---|---|---|---|
| | Formulation 4 | Formulation 7 | Formulation 8 |
| 5 | 48.7 | 46.5 | 56.3 |
| 10 | 69.7 | 66.3 | 74.5 |
| 15 | 84.5 | 76.5 | 84.0 |
| 30 | 91.6 | 86.1 | 91.4 |
| 45 | 97.4 | 89.7 | 94.2 |
| 60 | 101.0 | 95.8 | 97.2 |

From the above results, it was shown that low-substituted hydroxypropylcellulose at all three levels could achieve a drug dissolution rate of over 80% within 30 minutes. As the amount of the disintegrant increased, the drug dissolution was accelerated. To ensure rapid disintegration of the tablets and considering cost factors, the amount of the disintegrant needed to be controlled.

### 2.3 Selection of lubricant type and proportion

Commonly used lubricants for tablets, such as magnesium stearate, sodium stearyl fumarate, talc, and colloidal silicon dioxide, were chosen. The influence of lubricant type and proportion on the tablets of the compound of formula (I) was subsequently investigated, with the appearance, hardness, friability, and dissolution of the finished tablets serving as screening parameters. Wherein, microcrystalline cellulose and pregelatinized starch were used as the fillers, and low-substituted hydroxypropylcellulose was used as the disintegrant. The specific formulations are shown in Table 5 below.

**Table 5: Selection of lubricant types and proportions and the results**

| Component | Formulation 9 | Formulation 10 | Formulation 11 | Formulation 12 | Formulation 13 |
|---|---|---|---|---|---|
| Compound of formula (I) | 2 mg | 2 mg | 2 mg | 2 mg | 2 mg |
| Microcrystalline cellulose | 100 mg | 100 mg | 100 mg | 100 mg | 100 mg |
| Pregelatinized starch | 22 mg | 22 mg | 22 mg | 22 mg | 22 mg |
| Low-substituted hydroxypropylcellulose | 5 mg | 5 mg | 5 mg | 5 mg | 5 mg |
| Magnesium stearate | 1 mg | 0 | 0 | 0 | 0 |
| Sodium stearyl fumarate | 0 | 1 mg | 0 | 0 | 0 |
| Talc | 0 | 0 | 1 mg | 0 | 0 |
| Magnesium stearate + colloidal silicon dioxide (mass ratio 1:1) | 0 | 0 | 0 | 1 mg | 0 |
| Magnesium stearate + colloidal silicon dioxide (mass ratio 1:2) | 0 | 0 | 0 | 0 | 1 mg |

| Test Items | | | | | |
|---|---|---|---|---|---|
| Appearance | Pass | Pass | Cracking | Pass | Sticking |
| Hardness | 7.12 kg | 7.23 kg | 5.22 | 7.18 kg | 7.01 |
| Friability | 0.16% | 0.15% | Failed | 0.26% | 0.83% |

The compound of formula (I) in the proportions set forth in the formulations and the excipients listed in the above table were mixed homogeneously and compressed into tablets. The relevant parameters of the tablets were then tested. Among them, Formulations 11 and 13 were not further studied due to cracking and sticking, respectively. Formulations 9, 10, and 12 were selected for further dissolution evaluation. The tablets were subjected to dissolution testing at 37°C and 50 rpm using 500 mL of the pH 4.5 acetate buffer as the dissolution medium, with sampling at 5, 10, 15, 30, 45, and 60 minutes for determination of drug dissolution.

**Table 6 Dissolution test results of Formulations 9, 10, and 12**

| Time (min) | Dissolution rate (%) | | |
|---|---|---|---|
| | Formulation 9 | Formulation 10 | Formulation 12 |
| 5 | 49.2 | 46.3 | 48.7 |
| 10 | 70.2 | 69.7 | 69.2 |
| 15 | 85.6 | 77.1 | 84.1 |
| 30 | 92.5 | 89.8 | 91.9 |
| 45 | 98.3 | 96.4 | 97.9 |
| 60 | 100.8 | 99.5 | 100.1 |

From the comparison of the dissolution test results of the above formulations, Formulations 9 and 12 exhibited favorable dissolution profiles.

Based on the screening described above, it was found that the tablets of the compound of formula (I) prepared with magnesium stearate as a lubricant possessed hardness conforming to the requirements and demonstrated favorable dissolution behavior.

In summary, the intermediate powders of Formulations 4 and 12 exhibited good flowability and favorable mixing uniformity; and after compression, the hardness meets the requirements, and the amount of the disintegrants are moderate, so as to ensure rapid disintegration of the tablets. Therefore, Formulations 4 and 12 are the preferred formulations.

**Conclusion:** The study results of the aforementioned different formulations demonstrate that, with respect to the selection of excipients, magnesium stearate can significantly enhance the solubility of the drug and improve the dissolution of the tablets, as compared to talc.

### 2.4 Component proportions of the formulations

**Table 7: Formulations with different component proportions (mg/tablet and wt%)**

| Component | Formulation 14 | | Formulation 15 | | Formulation 16 | | Formulation 17 | | Formulation 18 | | Formulation 19 | | Formulation 20 | | Formulation 21 | | Formulation 22 | |
|---|---|---|---|---|---|---|---|---|---|---|---|---|---|---|---|---|---|---|
| | mg | % | mg | % | mg | % | mg | % | mg | % | mg | % | mg | % | mg | % | mg | % |
| Compound of formula (I) | 2 | 1.54% | 3 | 2.24% | 1.7 | 1.41% | 2.1 | 1.58% | 1.65 | 1.59% | 2.1 | 1.53% | 1.48 | 1.54% | 2.3 | 1.53% | 0.5 | 0.50% |
| Microcrystalline cellulose | 100 | 76.92% | 103 | 76.75% | 93 | 76.99% | 95 | 71.37% | 80 | 77.33% | 105 | 76.59% | 74.5 | 77.46% | 114.5 | 76.03% | 79 | 79.00% |
| Pregelatinized starch | 22 | 16.92% | 22 | 16.39% | 20.5 | 16.97% | 30 | 22.54% | 17 | 16.43% | 23 | 16.78% | 16 | 16.64% | 25 | 16.60% | 16.4 | 16.40% |
| Low-substituted hydroxypropylcellulose | 5 | 3.85% | 5.2 | 3.87% | 4.7 | 3.89% | 4.5 | 3.38% | 4 | 3.87% | 6 | 4.38% | 3.5 | 3.64% | 5.8 | 3.85% | 3.9 | 3.90% |
| Magnesium stearate | 1 | 0.77% | 1 | 0.75% | 0.9 | 0.75% | 1.5 | 1.13% | 0.8 | 0.77% | 1 | 0.73% | 0.7 | 0.73% | 3 | 1.99% | 0.2 | 0.20% |
| Total amount | 130 | 100% | 134.2 | 100% | 120.8 | 100% | 133.1 | 100% | 103.45 | 100% | 137.1 | 100% | 96.18 | 100% | 150.6 | 100% | 100 | 100% |

### Dissolution Analysis

According to Method II (for dissolution) of General Chapter of the Chinese Pharmacopoeia (2015 Edition, Part II), the dissolution profile of the drug was determined at 5 min, 10 min, 15 min, 30 min, 45 min, and 60 min using 500 mL of the pH 4.5 acetate buffer as the dissolution medium. The determination results are shown in Table 8.

**Table 8: Dissolution test results (%) of Formulation 14-Formulation 22**

| Time (min) | Formulations | | | | | | | | |
|---|---|---|---|---|---|---|---|---|---|
| | 14 | 15 | 16 | 17 | 18 | 19 | 20 | 21 | 22 |
| 5 | 49.5 | 30.2 | 50.8 | 45.3 | 51.1 | 30.5 | 51.6 | 31.1 | 58.3 |
| 10 | 70.3 | 45.1 | 65.9 | 57.3 | 67.3 | 66.2 | 66.7 | 65.8 | 71.2 |
| 15 | 85.8 | 52.3 | 90.2 | 65.6 | 89.9 | 90.6 | 91.1 | 91.1 | 95.2 |
| 30 | 92.7 | 67.5 | 95.6 | 78.2 | 98.7 | 96.2 | 98.5 | 96.4 | 99.3 |
| 45 | 98.5 | 85.4 | 99.2 | 86.3 | 99.6 | 98.8 | 99.9 | 97.9 | 100.1 |
| 60 | 101.1 | 91.7 | 100.8 | 91.6 | 100.2 | 100.2 | 101.3 | 100.6 | 102.3 |

From the comparison of the dissolution test results of the formulations above, the dissolution profiles of the pharmaceutical compositions with the proportions selected according to the present invention are better.

Through the screening described above, it was found that the tablets of the compound of formula (I) obtained according to the proportions of the present invention possessed hardness conforming to the requirements and demonstrated favorable dissolution behavior.

### 2.5 Particle size screening

The particle size of the active pharmaceutical ingredient of the compound of formula (I) may affect the disintegration and dissolution of the formulation. Meanwhile, as the present product has a low dosage strength (2 mg/tablet), excessive differences in particle size may readily result in nonuniform mixing and failure to meet content uniformity requirements. Therefore, the active pharmaceutical ingredient of the compound of formula (I) were subjected to sieving, and the active pharmaceutical ingredient with different particle sizes in the formulation-specified amount were taken, mixed with excipients, and compressed into tablets, and the hardness and dissolution were employed as evaluation parameters for investigation.

**Table 9: Formulation composition:**

| Component | Process 1 | Process 2 | Process 3 | Process 4 |
|---|---|---|---|---|
| Particle size D90 (µm) of active pharmaceutical ingredient | 11.58 | 8.22 | 5.14 | 48.73 |
| Compound of formula (I) | 2 mg | 2 mg | 2 mg | 2 mg |
| Microcrystalline cellulose | 100 mg | 100 mg | 100 mg | 100 mg |
| Pregelatinized starch | 22 mg | 22 mg | 22 mg | 22 mg |
| Low-substituted hydroxypropylcellulose | 5 mg | 5 mg | 5 mg | 5 mg |
| Magnesium stearate | 1 mg | 1 mg | 1 mg | 1 mg |
| Hardness | 7.38 kg | 7.26 kg | 8.28 kg | 8.14 kg |

The dissolution profiles of the tablets of the compound of formula (I) prepared by Processes 1, 2, 3 and 4 were determined at 37°C and 50 rpm using 500 mL of the pH 4.5 acetate buffer as the dissolution medium at 5, 10, 15, 30, 45 and 60 minutes. The determination results are shown in Table 10.

**Table 10 Dissolution test results of the tablets obtained from Processes 1, 2, 3, and 4**

| Time (min) | Process 1 | | Process 2 | | Process 3 | | Process 4 | |
|---|---|---|---|---|---|---|---|---|
| | Dissolution rate (%) | RSD (%) | Dissolution rate (%) | RSD (%) | Dissolution rate (%) | RSD (%) | Dissolution rate (%) | RSD (%) |
| 5 | 36.3 | 1.7 | 38.6 | 5.0 | 43.2 | 12.0 | 26.7 | 7.7 |
| 10 | 62.6 | 1.6 | 64.5 | 2.4 | 70.6 | 9.9 | 46.3 | 9.2 |
| 15 | 76.3 | 1.1 | 79.6 | 2.0 | 84.8 | 7.8 | 61.2 | 8.1 |
| 30 | 91.2 | 3.3 | 92.7 | 2.9 | 93.3 | 6.3 | 91.6 | 6.4 |
| 45 | 98.6 | 1.6 | 96.7 | 2.4 | 97.1 | 5.7 | 95.1 | 5.5 |
| 60 | 100.8 | 1.0 | 97.2 | 0.9 | 100.3 | 2.1 | 100.2 | 3.7 |

*f*2* represents the value when comparing Process 2 or Process 3 with Process 1 or Process 2; RSD refers to the relative standard deviation, wherein the RSD of less than 5% is considered to meet the requirement.

The results indicate that, in the comparison of the dissolution profiles of the tablets comprising the compound of formula (I) prepared from the active pharmaceutical ingredients having different particle size ranges in the pH 4.5 acetate buffer, the dissolution endpoint test results at the same time under the four process conditions were similar, all meeting the dissolution quality acceptance criteria. However, it was observed from the dissolution profiles that as the particle size decreased (as in the conditions of Process 2 and Process 3), the initial dissolution rate of the tablets became progressively faster, whereas under conditions of larger particle sizes (such as in Process 4), the initial dissolution rate of the tablets was significantly slower. Furthermore, the RSD values for Process 3 and Process 4 were notably higher, indicating that considerable differences in dissolution and non-uniformity. Taking all factors into consideration, controlling the particle size within the range between Process 1 and Process 2 can satisfactorily meet the requirements for clinical studies of relevant parameters such as Cmax and AUC, and also demonstrates certain advantages in terms of mass uniformity and stability. Therefore, it is determined that the preferred particle size range for the micronized active pharmaceutical ingredient of the compound of formula (I) is a D90 of 8-12 µm. To prevent agglomeration, the material should be passed through an 80-mesh sieve prior to manufacturing and charging.

### 2.6 Screening for mixing time

Taking Formulation 14 as an example, the specification of this product is 2 mg, with a tablet weight of 130 mg, and the active pharmaceutical ingredient accounts for 1.5%. The mixing uniformity directly affects the content uniformity of the tablets of the compound of formula (I). Direct mixing of the drug with excipients at formulation-specified proportions makes it difficult to achieve uniform mixing. It is proposed to adopt geometric addition for mixing this product using a mixer, and the mixing time is screened. The specific processes are shown in Table 11.

**Table 11 Process screening for mixing time**

| | Process 5 | Process 6 | Process 7 | Process 8 | Process 9 |
|---|---|---|---|---|---|
| Process Description | 1. The formulated amount of the compound of formula (I) and low-substituted hydroxypropylcellulose were taken and mixed using a three-dimensional mixer at 30 rpm for 5 min; | The mixing time for steps 1, 2, and 3 is 10 min, with the remaining step parameters being identical to those in Process 4. | The mixing time for steps 1, 2, and 3 is 15 min, with the remaining step parameters being identical to those in Process 4. | The mixing time for steps 1, 2, and 3 is 20 min, with the remaining step parameters being identical to those in Process 4. | The mixing time for steps 1, 2, and 3 is 25 min, with the remaining step parameters being identical to those in Process 4. |
| | 2. the formulated amount of pregelatinized starch was added thereto and mixed at 30 rpm for 5 min; | | | | |
| | 3. microcrystalline cellulose was added and mixed at 30 rpm for 5 min; | | | | |
| | 4. magnesium stearate was added and mixed at 30 rpm for 5 minutes. | | | | |

Samples were taken from three corners and from the upper and lower parts at the center of the container. The content was determined according to the content determination method, to calculate the mixing uniformity for evaluating the mixing time. The results are summarized in Table 12.

**Table 12 Determination results of intermediate powders obtained from Process 5-Process 9**

| Sample | Process 5 | Process 6 | Process 7 | Process 8 | Process 9 |
|---|---|---|---|---|---|
| Content (%) | 49.2 | 81.4 | 98.7 | 99.1 | 98.4 |
| | 45.1 | 86.0 | 84.6 | 103.0 | 97.0 |
| | 53.4 | 88.5 | 101.6 | 103.2 | 99.5 |
| | 49.5 | 81.3 | 95.7 | 98.5 | 97.9 |
| | 54.2 | 79.8 | 108.6 | 103.7 | 99.9 |
| Average content (%) | 50.3 | 83.4 | 97.8 | 101.5 | 98.6 |
| RSD (%) | 7.3 | 4.4 | 9.0 | 2.4 | 1.2 |
| Angle of repose | 42.1° | 41.8° | 40.9° | 40.1° | 38.2° |

From the results, it can be seen that the angle of repose of the intermediate powder under different mixing times met the requirements, and there was a decreasing trend in the angle of repose with increasing mixing time; the content at different parts of the intermediate powder was low when the mixing time was 5 min-10 min after the addition of each excipient; when the mixing time was 15 min, the RSD value was greater than 5%, which did not meet the requirements; when the mixing time was 20 min and 25 min, i.e., for Process 8 and Process 9, the RSD values were less than 5%, and the mixing uniformity met the requirements. Therefore, the mixing time range of 20 min-25 min was selected for addition of excipients other than magnesium stearate.

Since magnesium stearate has strong hydrophobicity, the mixing time for magnesium stearate was screened. The specific processes are shown in Table 13 below.

**Table 13 Optimization of lubricants mixing time**

| | Process 10 | Process 11 |
|---|---|---|
| Process Description | The mixture from Process 7 was taken, and added with the formulated amount of magnesium stearate, followed by mixing using a three-dimensional mixer at 30 rpm for 5 min. | The mixture from Process 7 was taken, and added with the formulated amount of magnesium stearate, followed by mixing using a three-dimensional mixer at 30 rpm for 5 min. |

Samples were taken from three corners and from the upper and lower parts at the center of the container. The content was determined according to the content determination method, to calculate the mixing uniformity for evaluating the mixing time. The results are summarized in Table 14.

**Table 14 Determination results of the mixed powders from Process 10-Process 11**

| Sample | Process 10 | Process 11 |
|---|---|---|
| | 103.0 | 100.3 |
| | 99.8 | 100.0 |
| Content (%) | 101.8 | 100.7 |
| | 101.8 | 101.2 |
| | 102.5 | 101.5 |
| Average content (%) | 101.8 | 100.7 |
| RSD (%) | 1.2 | 0.6 |
| Angle of repose | 40.5° | 40.3° |

As can be seen from the results, when magnesium stearate was added and then mixed for 5 min-10 min, the RSD value was less than 5%, and both the mixing uniformity and the angle of repose met the requirements. Therefore, the mixing time range of 5 min-10 min was selected for the addition of magnesium stearate.

Through further research, with respect to the geometric dilution method, the pharmaceutical compositions of the compound of formula (I) can be prepared by the following method:
1) Micronizing the active pharmaceutical ingredient to provide Material 1 with a D90 in the range of 8-12 µm;
2) Mixing Material 1 with pregelatinized starch by geometric dilution to provide Mixture 1, wherein Material 1 and pregelatinized starch are first mixed at a ratio of 1:2, followed by mixing the obtained mixture with pregelatinized starch by geometric dilution;
3) Thoroughly mixing Mixture 1 with microcrystalline cellulose and low-substituted hydroxypropyl cellulose to provide Mixture 2;
4) Adding magnesium stearate to Mixture 2 and mixing for 5-10 minutes to provide the final blended mixture;
5) Compressing the final blended mixture using a die to form tablets having a thickness of 3-5 mm.

### Example 1

**Table 15 Composition of Pharmaceutical Composition 1**

| Component | Amount | |
|---|---|---|
| | mg | % |
| Compound of formula (I) | 2.0 | 1.54 |
| Microcrystalline cellulose (M102) | 100.0 | 76.92 |
| Pregelatinized starch (STARCH 1500) | 22.0 | 16.92 |
| Low-substituted hydroxypropylcellulose (LH-21) | 5.0 | 3.85 |
| Magnesium stearate | 1.0 | 0.77 |
| Total weight | 130.0 | 100 |
| Solvents used in the process and finally removed | None | |

Scale-up studies were conducted for 3 batches of products, with batch sizes of 3,000 tablets/batch, 3,000 tablets/batch, and 10,000 tablets/batch, respectively, to achieve the following objectives:
To investigate the main steps of the production process and further optimize the process conditions;
To determine the equipment and production methods suitable for commercial manufacturing, ensuring product quality and reproducibility after process scale-up.

**Table 16-1 Summary of manufacturing process changes**

| Laboratory-scale process | Pilot-scale process | Major changes | Supporting evidence |
|---|---|---|---|
| The active pharmaceutical ingredient was sieved through an 80-mesh sieve, and the excipients were sieved through a 60-mesh sieve to remove any lumps, yielding uniform powders. | The active pharmaceutical ingredient was sieved through an 80-mesh sieve, and the excipients were sieved through a 60-mesh sieve to remove any lumps, yielding uniform powders. | No change | No change |
| According to the preparation method using the geometric dilution method described in Section 2.6 of Preparation Example 2, the formulated amounts of each component were weighed, sieved, and mixed homogeneously. | According to the mixing manner selected in Section 2.6 of Preparation Example 2 and the preparation method using the geometric dilution method described in Section 2.6, the formulated amount of each component was weighed and mixed homogeneously using a mixer, wherein Intermediate 1 was sampled (5 portions) from three corners and from the upper and lower parts at the center, respectively, for determining the mixing uniformity. | The materials were mixed using a mixer, and intermediate samples were taken for analysis. | During the pilot process, the amount of the sample was increased, and thus mixing was performed using a mixer, and intermediate analysis was added. |
| Tablet compression was performed using capsule-shaped punches. Samples were taken and tested for hardness and weight variation, and the results were found to meet the requirements. | Tablet compression was performed using capsule-shaped punches. Samples were taken and tested for hardness and weight variation, and the results were found to meet the requirements. | No change | No change |
| Packaged in polyvinyl chloride bags. | The product was packaged in oral solid medicinal high-density polyethylene bottles, with a packaging count of 30 tablets per bottle. Considering the hygroscopicity of the excipients, a desiccant was placed inside the bottle, which was then sealed to obtain the finished product. | -- | - |

**Table 16-2 Analytical results of quality control for three batches of samples**

| Batch No. | Scale (tablets) | Yield (%) | Active ingredient content (%) | Dissolution rate (%) |
|---|---|---|---|---|
| Batch 1 | 3000 | 87.6 | 101.9 | 92.4 |
| Batch 2 | 3000 | 88.0 | 101.7 | 92.8 |
| Batch 3 | 10000 | 90.8 | 102.1 | 91.3 |

Yield refers to the ratio of actual output to theoretical batch size; yield investigation concerns the consistency and stability of the process during production. High yield indicates good process control and relatively better quality assurance.

### Comparative Example

**Table 17 Composition of Pharmaceutical Composition 2**

| Component | Amount | |
|---|---|---|
| | mg | % |
| Compound of formula (I) | 2.0 | 1.55 |
| Microcrystalline cellulose (M102) | 101.0 | 77.69 |
| Lactose | 21.0 | 17.15 |
| Croscarmellose sodium | 5.0 | 3.85 |
| Magnesium stearate + Colloidal silicon dioxide (mass ratio 1:1) | 1.0 | 0.77 |
| Total weight | 130.0 | 100 |

The active pharmaceutical ingredient was sieved through an 80-mesh sieve, and the excipients were sieved through a 60-mesh sieve to remove any lumps, yielding uniform powders.

The compound of formula (I) in a formulated amount was weighed and added by geometric addition to croscarmellose sodium, lactose, and microcrystalline cellulose in formulated amounts, and mixed uniformly by sieving; then magnesium stearate + colloidal silicon dioxide (1:1) was added and mixed uniformly by sieving; and capsule-shaped punches were used for tableting. Samples were taken to test the hardness and weight variation of this product, and the results both met the requirements. The tablets were packaged in polyvinyl chloride bags.

### Effect Example 1

### High humidity test

Tablet samples from different Examples were taken and subjected to impurity determination and dissolution tests under high humidity conditions of 25°C and 90%RH at Day 0, Day 5, Day 10, and Day 30. The determination results are as follows:

**Table 18 Study results of high humidity testing for tablets**

| Test items | Acceptance criteria | Sample | Stability under high humidity conditions of 25°C and 90%RH | | | |
|---|---|---|---|---|---|---|
| | | | Day 0 | Day 5 | Day 10 | Day 30 |
| Characters | White or off-white tablets | Example 1 | White | White | White | White |
| | | Comparative Example | White | White | White | off-white |
| Total impurities | ≤2.0% | Example 1 | 0.54 | 0.54 | 0.53 | 0.55 |
| | | Comparative Example | 0.55 | 0.55 | 0.54 | 0.52 |
| Active ingredient content | 90.0%~110.0% | Example 1 | 101.6 | 101.8 | 102.3 | 102.5 |
| | | Comparative Example | 99.7 | 99.2 | 99.6 | 99.8 |
| Dissolution rate | >85% | Example 1 | 96.5 | 97.2 | 95.8 | 95.6 |
| | | Comparative Example | 89.2 | 86.1 | 83.6 | 82.4 |
| Friability | ≤1% | Example 1 | 0.16 | 0.17 | 0.15 | 0.17 |
| | | Comparative Example | 0.35 | 0.41 | 0.32 | 0.42 |
| Hardness | 4~8KGS | Example 1 | 6.32 | 6.59 | 6.43 | 6.41 |
| | | Comparative Example | 4.58 | 4.82 | 4.21 | 4.05 |

**Conclusion:** The results of the above studies of the different formulations and processes of the Examples indicate that, Example 1, which selected a formulation mainly composed of insoluble excipients, showed relatively small changes in terms of characters and impurities compared to other Examples under relatively high humidity conditions, and the quality of the finished product was more stable.

### Photostability test

30 mg of tablet samples from Example 1 were taken and subjected to impurity determination and dissolution tests under light exposure conditions at Day 0, Day 5, Day 10, and Day 30. The determination results are as follows:

**Table 19 Study Results of Photostability testing for tablets of Example 1**

| Test item | Acceptance criteria | Sample | Photostability | | | |
|---|---|---|---|---|---|---|
| | | | Day 0 | Day 5 | Day 10 | Day 30 |
| Characters | White or off-white tablets | Example 1 | White tablets | White tablets | White tablets | White tablets |
| Total impurities | ≤2.0% | Example 1 | 0.55 | 0.53 | 0.56 | 0.58 |
| Active ingredient content | 90.0%~110.0% | Example 1 | 102.11 | 100.94 | 101.16 | 101.82 |
| Dissolution rate | ≥80% | Example 1 | 91.3 | 90.6 | 91.7 | 90.9 |

### High temperature test

Tablet samples from Example 1 were taken and subjected to impurity determination and dissolution tests under high temperature conditions of 60°C at Day 0, Day 5, Day 10, and Day 30. The determination results are as follows:

**Table 20 Study Results of high temperature stability testing for the tablets of Example 1**

| Test item | Acceptance criteria | High temperature stability | | | |
|---|---|---|---|---|---|
| | | Day 0 | Day 5 | Day 10 | Day 30 |
| Characters | White tablets | White tablets | White tablets | White tablets | White tablets |
| Total impurities | ≤2.0% | 0.55 | 0.55 | 0.54 | 0.53 |
| Active ingredient content | 90.0%~110.0% | 102.11 | 101.53 | 102.49 | 101.76 |
| Dissolution rate | ≥80% | 91.3 | 92.4 | 91.5 | 90.8 |

### Accelerated experiment

Three batches of tablet samples from Example 1 were taken and subjected to impurity determination and dissolution tests at day 0, and at 1, 2, 3, and 6 months under accelerated conditions (40°C±2°C, 75%±5%RH);

The determination results are as follows:

**Table 21 Study results of accelerated testing for the tablets of Example 1**

| Test item | Acceptance criteria | Time (month) | | | | |
|---|---|---|---|---|---|---|
| | | 0 | 1 | 2 | 3 | 6 |
| Characters | White to off-white tablets | White tablets | White tablets | White tablets | White tablets | White tablets |
| Total impurities | ≤2.0% | 0.54 | 0.59 | 0.50 | 0.50 | 0.44 |
| Active ingredient content | 90.0%~110.0% | 101.9 | 102.4 | 101.7 | 102.0 | 100.5 |
| Dissolution rate | ≥80% | 92.4 | 91.3 | 91.5 | 91.4 | 87.8 |
| Microbial Limits | Complies | Complies | / | / | Complies | Complies |

### Long-term experiment

Tablet samples from Example 1 were taken and subjected to impurity determination and dissolution tests at Day 0, and at 3 and 6 months under long-term storage conditions (25±2°C/60±5%RH). The determination results are as follows:

**Table 22 Study results of long-term testing for the tablets of Example 1**

| Test item | Acceptance criteria | Time (month) | | |
|---|---|---|---|---|
| | | 0 | 3 | 6 |
| Characters | White to off-white tablets | White tablets | White tablets | White tablets |
| Total impurities | ≤2.0% | ≤2.0% | 0.54 | 0.50 |
| Active ingredient content | 90.0%~110.0% | 90.0%∼110.0% | 101.9 | 100.4 |
| Dissolution rate | ≥80% | ≥80% | 92.4 | 90.7 |
| Microbial Limits | Complies | Complies | Complies | Complies |

Conclusion: It can be seen from the study results in Table 18-Table 22 above that the pharmaceutical composition of the present application (represented by Example 1) can meet the high standard quality requirements and has excellent stability.

### Effect Example 2: Pharmacokinetics following single administration in healthy Chinese subjects

Trial design: single-center, randomized, double-blind, placebo-controlled, dose-escalation, single administration. Number of subjects: Planned enrollment: 52 subjects, Final analysis: 51 subjects.

### Subject inclusion criteria:

Age 18 years ≤ age ≤ 40 years, healthy subjects, gender unrestricted;
Weight: Male ≥ 50 kg, female ≥ 45 kg; and 19 ≤ body mass index (BMI) ≤ 26 kg/m²;
No history of diseases of heart, liver, kidney, gastrointestinal, nervous system, mental disorders, and metabolic abnormalities, and no history of severe infection or severe injury;
No plan for pregnancy during the study period and within 3 months after the last dose, and agree to take reliable contraceptive measures;
Fully understand the purpose and requirements of this trial, voluntarily participate in the clinical trial and sign the written informed consent form, and willing to complete the entire trial process as required by the trial.

### Subject exclusion criteria:

Individuals with known history of allergy to the component of the tested drugs or similar drugs, history of allergic diseases, or allergic constitution;
Individuals with history of central nervous system, cardiovascular system, digestive system, respiratory system, urinary system, hematological system diseases, history of metabolic disorders, history of malignant tumors, or other history of diseases unsuitable for participating in clinical trials (such as history of psychiatric disorders);
Any surgical condition or disease that may significantly affect drug absorption, distribution, metabolism, and excretion, or any surgical condition or disease that may pose a hazard to subjects participating in the trial; such as history of gastrointestinal surgery (gastrectomy, gastroenteroanastomosis, enterectomy, etc.), urinary tract obstruction or dysuria, history of gastroenteritis, gastrointestinal ulcer, gastrointestinal bleeding, etc;
History of clinically significant electrocardiogram (ECG) abnormalities, or presence of the following electrocardiogram abnormalities at screening or baseline: PR interval > 220 ms, QRS complex duration > 120 ms, or QTcB > 450 ms;
Laboratory tests showing clinically significant abnormalities [a. laboratory tests of liver function such as aspartate aminotransferase (AST), alanine aminotransferase (ALT), alkaline phosphatase (ALP), or bilirubin , showing clinically significant abnormalities, suggesting liver disease or liver damage, or defined as exceeding 1.5 times the upper limit of normal; b. creatinine and blood urea nitrogen exceeding the upper limit of normal with clinical significance, or abnormal urine components with clinical significance (such as proteinuria, occult blood in urine, leukocytes in urine); c. clinically significant abnormalities in blood routine examination, such as anemia, decreased leukocyte count, significantly decreased platelet count];
Blood donation or blood loss ≥ 400 mL within 3 months prior to enrollment;
Use of any medication within 2 weeks prior to enrollment;
Pregnant or lactating women;
   Participation in other drug clinical trials within one month prior to enrollment;
History of drug abuse or positive urine test for drug abuse;
Current or past alcohol users (alcohol consumption exceeding 196 g per week, such as 360 mL beer or 45 ml spirits with 40% alcohol content, or 150 ml wine), or positive breath alcohol test;
Smoking ≥ 10 cigarettes per day;
Positive for hepatitis B surface antigen (HBsAg), hepatitis C virus antibody (HCV-Ab), syphilis antibody and HIV antibody (HIV-Ab);
Other factors that the investigator considers unsuitable for participating in the trial.

### Test drug:

Specifications: 0.5 mg/tablet, 1 mg/tablet, 2 mg/tablet, prepared according to the method of Example 1;
Dose: 0.5 mg, 1 mg, 2 mg, 4 mg, 8 mg, 12 mg.
Administration method: Single-dose, taken with an appropriate amount of water (240 mL).

### Control drug (placebo):

Specifications: 1 mg/tablet, 2 mg/tablet;
Dose: NA.

Administration method: Single-dose, taken with an appropriate amount of water (240 mL).

Evaluation indicators of pharmacokinetic parameters: Time to peak (Tmax), peak concentration (Cmax), area under the plasma concentration time curve from 0 to t (AUC₀₋ₜ), area under the plasma concentration time curve from 0 to infinity (AUC_{0-∞}), elimination rate constant (λz), half-life period (t_{1/2}), total clearance/bioavailability (CL/F), apparent volume of distribution/bioavailability (Vd/F).

Pharmacokinetic analysis: Pharmacokinetic parameters were calculated using the non-compartmental analysis (NCA) and Phoenix WinNonlin 7.0 data analysis software. Descriptive statistical analysis was performed on pharmacokinetic parameters, providing the number of subjects, arithmetic mean, standard deviation, minimum, median, maximum, and coefficient of variation (CV). Tmax was expressed as median (minimum - maximum). AUC and Cmax belong to a log-normal distribution. The geometric mean was also provided. The relationship between administered dose and drug plasma exposure was evaluated by plotting scatter plots of log-transformed pharmacokinetic parameters (AUC and Cmax) in relation to log dose. Furthermore, the Power model method was used to further explore the linear analysis of administered dose and drug plasma exposure.

### Pharmacokinetic results:

A single dose of the tablets of the compound of formula (I) was administered orally to healthy volunteers on empty stomach. The compound of formula (I) in the plasma reached the peak concentration at 0.75 h-1.5 h (median), and the mean plasma drug elimination half-life period was 14.2 h-32.1 h. When the administered doses were 1 mg, 2 mg, 4 mg, 8 mg, and 12 mg, the peak plasma drug concentrations were 1.19 ng/mL, 2.43 ng/mL, 8.61 ng/mL, 16.4 ng/mL, and 23.5 ng/mL, respectively, and the AUC_{0-∞} were 14.7 h·ng/mL, 29.8 h·ng/mL, 69.4 h·ng/mL, 135 h·ng/mL, and 220 h·ng/mL, respectively. The correlation and linear analysis of drug plasma exposure with the administered dose indicated that, within the range of 1 mg-12 mg, the β values for Cmax and AUC_{0-∞} of the compound of formula (I) were 1.22 and 1.08, respectively. Within the dose range of 1 mg-12 mg, Cmax and AUC_{0-∞} increased with increasing administered dose, and the increase ratio in AUC_{0-∞} was basically consistent with the dose increase ratio. The inter-individual variability of plasma drug exposure is around 30%.

Metabolite M4 in plasma reached the peak concentration in 1.5 h-1.75 h (median), and the mean plasma drug elimination half-life period was 2.88 h-7.19 h. At the administered dose of 1 mg-12 mg, the metabolite ratio MR_Cmax of M4 was 0.238 ng/mL-0.377 ng/mL, and MR_AUC was 0.153 h·ng/mL-0.220 h·ng/mL.

### Conclusion

A single dose of the tablets of the compound of formula (I) was administered orally to healthy volunteers on empty stomach. The compound of formula (I) in the plasma reached the peak concentration in 0.75 h-1.5 h (median), and the mean plasma drug elimination half-life was 14.2 h-32.1 h. Within the dose range of 1 mg-12 mg, Cmax and AUC_{0-∞} increased with increasing administered dose, and the increase ratio in AUC_{0-∞} was basically consistent with the dose increase ratio.

No SAE occurred within the dose range of 1 mg-12 mg, and no treatment-emergent adverse events (TEAEs) of Grade 3 or above occurred. Overall, the safety and tolerability of a single-dose administration of the tablets of the compound of formula (I) in healthy Chinese subjects were good, consistent with the safety risks suggested by preclinical studies of the tablets of the compound of formula (I) and the similar drug donepezil.

In summary, the tablets of the compound of formula (I) prepared in the present invention possess good in vivo efficacy, and the exposure shows a proportional relationship with the dose; the prepared tablets possess stable quality and good blend uniformity. After compression, the hardness of the tablet meets the requirements, and the dosages of disintegrants are appropriate, ensuring rapid disintegration of the tablets. The direct powder compression process used in the present invention is relatively simple, eliminating the need for granulation and drying, simplifying the process flow, improving production efficiency, and saving energy.

## Claims

1. A pharmaceutical composition comprising a compound of formula (I) or a pharmaceutically acceptable salt thereof, and a pharmaceutically acceptable excipient, wherein the pharmaceutically acceptable excipient is selected from one or more of a filler, disintegrant, and lubricant,

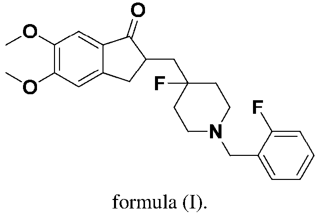

2. The pharmaceutical composition according to claim 1, **characterized in that**, in percent by weight, the pharmaceutical composition comprises 0.50 wt%-20.00 wt% of the compound of formula (I) or a pharmaceutically acceptable salt thereof.

3. The pharmaceutical composition according to claim 2, **characterized in that**, in percent by weight, the pharmaceutical composition comprises 0.50 wt%-5.00 wt% of the compound of formula (I) or a pharmaceutically acceptable salt thereof.

4. The pharmaceutical composition according to any one of claims 1-3, **characterized in that**, the pharmaceutical composition comprises the compound of formula (I) or a pharmaceutically acceptable salt thereof having a D90 particle size of 5 µm-50 µm.

5. The pharmaceutical composition according to claim 4, **characterized in that**, the pharmaceutical composition comprises the compound of formula (I) or a pharmaceutically acceptable salt thereof having a D90 particle size of 8 µm-12 µm.

6. The pharmaceutical composition according to any one of claims 1-5, **characterized in that**,
the filler is selected from pregelatinized starch and microcrystalline cellulose, and the filler accounts for 55.00 wt%-97.00 wt% of the weight of the pharmaceutical composition;
the disintegrant is selected from one or more of starch and derivatives thereof, low-substituted hydroxypropylcellulose, cross-linked polyvinylpyrrolidone, croscarmellose sodium, and cross-linked povidone, and the disintegrant accounts for 0.50 wt%-7.00 wt% of the weight of the pharmaceutical composition; or
the lubricant is selected from one or more of colloidal silicon dioxide, sodium stearyl fumarate, and magnesium stearate, and the lubricant accounts for 0.10 wt%-5.00 wt% of the weight of the pharmaceutical composition.

7. The pharmaceutical composition according to any one of claims 1-5, **characterized in that**,
the filler is selected from pregelatinized starch and microcrystalline cellulose, and the filler accounts for 90.00 wt%-96.00 wt% of the weight of the pharmaceutical composition;
the disintegrant is selected from one or more of starch and derivatives thereof, low-substituted hydroxypropylcellulose, cross-linked polyvinylpyrrolidone, croscarmellose sodium, and cross-linked povidone, and the disintegrant accounts for 2.00 wt%-5.50 wt% of the weight of the pharmaceutical composition; or
the lubricant is selected from one or more of colloidal silicon dioxide, sodium stearyl fumarate, and magnesium stearate, and the lubricant accounts for 0.20 wt%-2.00 wt% of the weight of the pharmaceutical composition.

8. The pharmaceutical composition according to any one of claims 1-5, **characterized in that**, the filler is a combination of microcrystalline cellulose and pregelatinized starch;
the disintegrant is selected from low-substituted hydroxypropylcellulose and/or croscarmellose sodium; or
the lubricant is selected from colloidal silicon dioxide and/or magnesium stearate.

9. The pharmaceutical composition according to any one of claims 1-5, **characterized in that**, the pharmaceutical composition comprises: 0.50 wt%-4.00 wt% of the compound of formula (I) or a pharmaceutically acceptable salt thereof; 65 wt%-97.00 wt% of a filler, wherein the filler is microcrystalline cellulose; 0.50 wt%-7.00 wt% of a disintegrant, wherein the disintegrant is selected from one or more of low-substituted hydroxypropylcellulose and croscarmellose sodium; and, 0.10 wt%-5.00 wt% of a lubricant, wherein the lubricant is selected from one or more of colloidal silicon dioxide, sodium stearyl fumarate, and magnesium stearate;
alternatively, the pharmaceutical composition comprises: 0.50 wt%-4.00 wt% of the compound of formula (I) or a pharmaceutically acceptable salt thereof; 65 wt%-97 wt% of a filler, wherein the filler is a combination of microcrystalline cellulose and pregelatinized starch; 0.50 wt%-7.00 wt% of a disintegrant, wherein the disintegrant is low-substituted hydroxypropylcellulose; and, 0.10 wt%-5.00 wt% of a lubricant, wherein the lubricant is sodium stearyl fumarate or magnesium stearate;
alternatively, the pharmaceutical composition comprises: 0.50 wt%-4.00 wt% of the compound of formula (I) or a pharmaceutically acceptable salt thereof; 65 wt%-97 wt% of a filler, wherein the filler is a combination of microcrystalline cellulose and pregelatinized starch; 0.50 wt%-7.00 wt% of a disintegrant, wherein the disintegrant is low-substituted hydroxypropylcellulose; and, 0.10 wt%-5.00 wt% of a lubricant, wherein the lubricant is a combination of colloidal silicon dioxide and magnesium stearate;
alternatively, the pharmaceutical composition comprises: 0.50 wt%-4.00 wt% of the compound of formula (I) or a pharmaceutically acceptable salt thereof; 65 wt%-97 wt% of a filler, wherein the filler is a combination of microcrystalline cellulose and pregelatinized starch; 0.50 wt%-7.00 wt% of a disintegrant, wherein the disintegrant is low-substituted hydroxypropylcellulose; and, 0.10 wt%-5.00 wt% of a lubricant, wherein the lubricant is magnesium stearate.

10. The pharmaceutical composition according to any one of claims 1-5, **characterized in that**,
the pharmaceutical composition comprises:
0.50wt%-2.50wt% of the compound of formula (I) or a pharmaceutically acceptable salt thereof;
92.00 wt%-96.00 wt% of a filler, wherein the filler is a combination of microcrystalline cellulose and pregelatinized starch;
2.00 wt%-4.50 wt% of a disintegrant, wherein the disintegrant is low-substituted hydroxypropylcellulose; and 0.20 wt%-2.00 wt% of a lubricant, wherein the lubricant is magnesium stearate.

11. The pharmaceutical composition according to any one of claims 1-5, **characterized in that**, the pharmaceutical composition is in the form of a tablet.

12. The pharmaceutical composition according to claim 11, **characterized in that**, the thickness of the tablet is 1 mm-10 mm.

13. The pharmaceutical composition according to claim 12, **characterized in that**, the thickness of the tablet is 3 mm-5 mm.

14. A method of preparing a pharmaceutical composition according to any one of claims 1-13, comprising:
micronization: micronizing an active pharmaceutical ingredient;
mixing by geometric dilution: mixing the micronized active pharmaceutical ingredient with at least a portion of a filler by geometric dilution;
final blending: mixing the mixture obtained from mixing by geometric dilution, with the remaining portion of the filler, a disintegrant, and a lubricant simultaneously and/or sequentially, to provide a final blended mixture;
compression: compressing the final blended mixture.

15. The method according to claim 14, comprising:
1) micronizing the active pharmaceutical ingredient is to obtain Material 1;
2) mixing Material 1 with at least a portion of a filler by geometric dilution, to provide Mixture 1;
3) mixing Mixture 1, with the remaining portion of the filler and a disintegrant at 20 rpm-40 rpm for 20 min-25 min, to provide a homogeneous Mixture 2;
4) adding a lubricant to Mixture 2 and mixing at 20 rpm-40 rpm for 5 min-10 min, to provide the final blended mixture;
5) compressing the final blended mixture using a die, to form tablets having a thickness of 1 mm-10 mm.

16. The method according to claim 14 or 15, **characterized in that**, the active pharmaceutical ingredient is micronized to a D90 particle size of 5 µm-50 µm.

17. The method according to claim 16, **characterized in that**, the active pharmaceutical ingredient is micronized to a D90 particle size of 8 µm-12 µm.

18. The method according to any one of claims 14-17, **characterized in that**, during mixing by geometric dilution, each mixing being conducted at 20 rpm-40 rpm for 20 min-25 min.

19. The method according to any one of claims 14-18, **characterized in that**, it forms tablets having a thickness of 1 mm-10 mm.

20. The method according to any one of claims 14-19, wherein the method comprises:
1) micronizing the active pharmaceutical ingredient to provide Material 1 with a D90 particle size of 8 µm-12 µm;
2) mixing Material 1 with one of the filler by geometric dilution, to provide Mixture 1, wherein each mixing is conducted at 20 rpm-40 rpm for 20 min-25 min;
3) mixing Mixture 1 with the remaining portion of the filler and a disintegrant at 20 rpm-40 rpm for 20 min-25 min, to provide Mixture 2;
4) adding a lubricant to Mixture 2 and mixing at 20 rpm-40 rpm for 5 min-10 min, to provide the final blended mixture;
5) compressing the final blended mixture using a die, to form tablets having a thickness of 3 mm-5 mm.

21. The method according to any one of claims 14-20, wherein the method comprises:
1) micronizing the active pharmaceutical ingredient to provide Material 1 with a D90 partical size of 8 µm-12 µm;
2) mixing Material 1 with pregelatinized starch by geometric dilution, to provide Mixture 1, in which Material 1 is initially mixed with pregelatinized starch at a ratio of 1:2, followed by mixing the obtained mixture with pregelatinized starch by geometric dilution, wherein each mixing is conducted at 20 rpm-40 rpm for 20 min-25 min;
3) mixing Mixture 1 with microcrystalline cellulose and low-substituted hydroxypropylcellulose at 20 rpm-40 rpm for 20 min-25 min, to provide Mixture 2;
4) adding magnesium stearate to Mixture 2 and mixing at 20 rpm-40 rpm for 5 min-10 min to provide the final blended mixture;
5) compressing the final blended mixture using a die, to form tablets having a thickness of 3 mm-5 mm.

22. Use of a pharmaceutical composition according to any one of claims 1-13, or a pharmaceutical composition prepared by the method according to any one of claims 14-21, in the preparation of a medicament for treating a nervous system disease associated with acetylcholinesterase.
